# EUROPEAN PATENT APPLICATION

(11) **EP 2 868 346 A1**
(43) Date of publication of application: **06.05.2015**
(21) Application number: 13797280.8
(22) Date of filing: 22.05.2013
(51) Int. Cl.: A61N 2/02

(54) **METHOD AND DEVICE FOR THE DESTRUCTION OF CELLS WITH UNCONTROLLED PROLIFERATION**

(30) Priority: 31.05.2012 ES 201200577
(71) Applicant: Investigaciones, Desarrollos e Innovaciones Tat Iberica, S.L., 28794 Guadalix de la Sierra (Madrid) (ES)
(72) Inventor: DÍAZ VALLE, Ricardo, 31190 Cizur Menor (Navarra) (ES); TEJADA SOLÍS, Sonia, 31190 Cizur Menor (Navarra) (ES); SEPÚLVEDA SÁNCHEZ, Juan Manuel, 28029 Madrid (ES); RODRÍGUEZ GONZÁLES, Virginia, 280 Madrid (ES); CASTAÑOS CANTOS, Ana, 28011 Madrid (ES); TORRES GÓMEZ, Maria, 28011 Madrid (ES); DEL CAMPO SÁNCHEZ, Ildefonso, 28007 Madrid (ES); TORRES JUNCO, Vicente, 28794 Guadalix de la Sierra (Madrid) (ES)
(74) Representative: Garcia-Cabrerizo y del Santo, Pedro Maria
(86) International application number: PCT/ES2013/000125
(87) International publication number: WO 2013/178839

(57) **Abstract**

The present invention relates to a method and device for the selective destruction of cells that with uncontrolled proliferation in an animal organism, with minimum damage to the surrounding tissue thereby and without needing to perform any kind of surgical operation on the patient: the method consists of the detection of the tumorous area (1) and the sequential and alternate application of at least two alternating magnetic fields with different directions to the treatment area or application until achieving the heating and destruction of the cells. The device used is, therefore, comprised of at least two electromagnets (11, 12) or at least two pairs of electromagnets (11-11', 12-12') all being located around the perimeter of a hypothetical circumference or sphere, with the electromagnets in opposing pairs where each opposing pair generates an electromagnetic field in one direction and orientation.

## Description

### Technical sector of the invention

The invention relates to a method and device for the selective destruction of cells with uncontrolled proliferation, or other types of undesirable cells, in an animal organism, or for causing a specific injury in living beings, organs, cells, viruses, etc. Said cells are selectively destroyed with minimum damage to the surrounding tissue and without needing to perform any kind of surgical operation on the patient.

Its field of application is:
a) It is aimed at human and veterinary medicine in the treatment of animal organisms.
b) Thermal engineering.

### Background of the invention

The detection of cancerous cells with uncontrolled proliferation is essential for the survival of a patient. The majority of malignant tumours cause metastasis: cells from the tumour tissue that separate and spread through an animal organism, this may be through the lymphatic or circulatory systems depending on the type of tumour and its location. These cells circulate until they attach to another place within the organism and continue to multiply causing new metastasis and new tumours. The only functional activity of these cells is to ensure their continued division; as a result they produce hyperactive cell clumps that affect vital organs by supplanting their organic tissue without performing their essential functions.

If a primary tumour is detected in time it is often possible to eliminate it using surgery, radiation therapy or chemical therapy or some combination of these treatments. Unfortunately the metastatic colonies are difficult to detect and eliminate and it is often impossible to successfully treat them all.

Even if treating the disease using chemotherapy or radiotherapy slows its advance it will not cure the disease and a patient's quality of life will suffer due to the severe side effects associated with the treatment.

Where the cancer has not spread the patient can be treated using surgery and radiotherapy or a combination of both techniques. This treatment is often effective in curing the disease. Where the metastasis is generalised it is only possible to use radiotherapy and this is rarely effective as the disease is widespread and the vital organs will have been affected. This makes surgery more complicated and dangerous. All of these factors have the cumulative effect of resulting in a short life expectancy for the patient.

The present invention proposes an alternative to the existing techniques for the elimination of highly active cell aggregations that are exhibiting uncontrolled cell proliferation (tumour cells). It permits their selective destruction without side effects and without needing to perform any kind of surgical operation on the patient and with the obvious advantages that this brings for expediting and improving patient recovery as well as providing a significant reduction in surgical costs.

### Description of the invention

The present invention relates, firstly, to a method to be followed for the destruction of cells that with uncontrolled proliferation, as well as other types of damaging or undesirable cells, through the application of electromagnetic fields, as explained below.

The other part of the present invention relates to a device that generates one or more electromagnetic fields, whose intensity, duration, direction and frequency of activation / deactivation can be determined using a software program installed in the device's microprocessor.

The present invention is based on the use of electromagnetic fields to bring about a specific orientation of polarised molecules that interact with the lines of the generated electromagnetic field. A polarised molecule that is subjected to a magnetic field will orientate itself to become positioned in the same direction as the line of this electromagnetic field.

If polarised molecules are subjected to the sequential, alternate application of electromagnetic fields this will give rise to changes in the orientation of these molecules causing them rotate or move.

Therefore, the sequential application of alternate electromagnetic fields to the cells to be destroyed will cause a change in the orientation of the polarised molecules present in the area affected by the fields. This will bring about an increase in temperature due to the movement and friction of the turning molecules. The method relating to the present invention involves the sequential and repeated application of at least two electromagnetic fields to the treatment area. These fields will have different directions so that the molecules that are orientated by the first field will turn and change their orientation again under the influence of the second field. Once they have changed their orientation the initial field is applied again so that the molecules change orientation again. This is repeated until friction caused by the movement of the polarised molecules (bipolar) in relation to their surroundings brings about an increase in temperature in the cells. This will, in turn, provoke the thermal destruction of the cells in question.

The magnetic field lines must therefore pass through the cells or cellular accumulation that has to be destroyed in order to produce this sequential change in direction. In order to do this it is first necessary to form or create a closed three-dimensional space that relates to the intersection of at least two electromagnetic fluxes or fields arising from different spatial locations. This will create a predetermined volume or space in the area of interest, that is, in the area containing the cells with uncontrolled proliferation or tumours or a location where a specific predetermined injury or industrial application is required. The current from each of the magnetic fields will pass through the space or volume formed by the intersection of the magnetic fields (specific closed intersection space). Therefore, any bipolar structure located inside that intersection space will be subjected to the actions produced by each of the magnetic currents passing through the space.

The electromagnetic current is generated when electrical current flows through an electromagnet "containing air core coils". If an electrical current flows then a vectoral electromagnetic field is generated that has direction and orientation and intensity and it emanates from the interior of each coil or from the electromagnet. The activation and deactivation of the different coils is coordinated so that one of the coils is active when the other is not, meaning that the intersection space is subjected to alternate electromagnetic currents generated by the activated coil.

The dwell time of each electromagnetic field acting on the intersection space is configurable, depending on the required temperature the tumour cells are to be subjected to.

The maximum temperature to be reached in the focal area is also configurable by temporarily interrupting the activation / deactivation cycles if a set temperature is exceeded. Or the activation / deactivation cycle of the coil can be initiated if the temperature in the focal area drops below a predetermined value.

The temperature within the focal area can be detected using infrared techniques and it can be configured by setting a desired maximum or minimum temperature or level of thermal dose during the exposure time. This will allow selective control of the cells to be treated, so that the cells that first reach this temperature are treated for longer thereby provoking their death or alteration, yielding cellular structures that are destroyed by the immunological system. This will mean that the cells that take longer to reach the programmed temperature will be unaffected by the lethal thermal effects and by the effect of remaining for any time at an elevated temperature, which could cause their death.

To enhance the performance of the magnetic fields on the tumour cells they can be combined or associated with paramagnetic or even ferromagnetic materials that will be more strongly affected by the electromagnetic field. This will allow the tumour cells to be differentiated from those cells that do not have this association. This will, in turn, allow the non-tumour cells that are surrounded or enveloped by harmful cells to avoid the thermal effects caused by the changes in electromagnetic field. That is, for the same exposure time, there will be a much more rapid temperature increase in the tumour cells that are associated with materials that enhance their polar structure than will be seen in the non-tumorous cells. This means that selective treatment can be given for tumour cells located within the electromagnetic fields' intersection space.

The thermal effect will be similar to that produced in ultrasound techniques, although with a more precise focal effect given that the field of action (interaction space) is related to the diameter of the coils and these can easily be made with relatively small diameters (coils of around 2 mm in diameter or even smaller would be sufficient to treat small areas). Moving or displacing the axes of the magnetic circuits can decrease the intersection area until it is practically selective.

The electromagnetic fields are applied sequentially and alternately in different directions for the number of times and duration necessary to achieve the required increase in temperature that will produce cell death.

The preferred frequency of the electromagnets' activation / deactivation cycles, that is, the frequency or number of times a second the electromagnetic field is repeatedly applied to the treatment area, will be within the short wave frequencies (0.5 GHz to 10 GHz) or more specifically at industrial frequencies of around 915 MHz. The frequency will depend on the volume and area of the reference polarity, as well as on its molecular structure and the intersection angle of the direction of the electromagnetic fields. The activation frequency (rate of activation / deactivation) of the fields is related to the intersection angle of the electromagnets' axes. If a material has to vibrate at 2.45 GHz in order to cause its heating and the electromagnets' axes form an angle of 90° the frequency (activation / deactivation) will be multiplied by 2, which is the result of dividing 180 by 90. The lower the angle, the greater the frequency.

The distance at which the influence of each of the coils' fields can be physically noted will depend on the intensity of the current flowing through the coil, as well as on their number and on the characteristics of the material that the generated electromagnetic circuit passes through.

The described method for using the present invention, therefore, permits the destruction of tumour cells by heat generated by the sequential and alternate application of electromagnetic fields to those tumorous cells for a specified time.

Each electromagnetic field is generated by an electromagnet, or preferably, by a pair of electromagnets with their opposite poles facing each other so that the field lines generated between a pair of electromagnets pass through the treatment area (tumorous molecular aggregations). It is preferable that the electromagnetic field is generated by two electromagnets as this improves the focussing of the field lines and allows them to be better directed through the treatment area. However, only one electromagnet can be used as long as the field lines generated by this electromagnet pass through the treatment area.

In order to do this, it is first necessary to detect the area of the organism where the tumours or cells with uncontrolled proliferation to be destroyed are located.

To summarise, the present method for the selective destruction of cells in an animal organism is comprised of the following steps:
a) Detection of the cells with uncontrolled proliferation in the animal organism of interest. This detection can be performed using any state of the art technique: using a contrast agent, radiography, resonance, scanning techniques, etc.
b) Sequential and alternate application of at least two magnetic fields with different directions to the treatment area to achieve the heating and destruction of the cells, where each electromagnetic field is generated by at least one electromagnet, but preferably with a pair of electromagnets with their opposite poles facing as this allows better focussing of the flow lines.

The tumorous cells, polyps, warts, etc. can be labelled or associated with paramagnetic or even ferromagnetic materials before the sequential and alternate application of at least two magnetic fields in order to enhance the action of the electromagnetic fields.

This technique involves the application of magnetic fields with differing directions specifically to tumorous cells or cellular aggregations thereby eliminating the tumorous cells without affecting the surrounding tissue and ensuring that the patient suffers minimal side effects.

The device employed to carry out this method is comprised of at least two electromagnets that generate electromagnetic fields in different directions but arranged so that the lines of the magnetic fields intersect in a predetermined spatial area (intersection space). However, it is preferable that the device has at least four electromagnets arranged around the perimeter of a hypothetical circumference or sphere, the electromagnets face each other in pairs so that two opposing electromagnets generate a singe electromagnetic field in a predetermined direction but where the direction generated by one pair is opposite to that generated by the other pair of opposing magnets. Two opposing electromagnets are located so that their opposing poles face each other in order to generate an electromagnetic field between them. Generating the electromagnetic field by placing the two opposing electromagnets opposite each other helps to focus the flux lines that must pass through the treatment area. Where a pair of electromagnets is used to generate an electromagnetic field in one direction the device will have an even number of electromagnets located around a hypothetical circumference or sphere if the magnets have the same physical characteristics (for instance they should have the same intensity of magnetic current), if not, there has to be an eccentric displacement depending on the differing characteristics. The magnets should oppose each other in pairs that generate an electromagnetic field focussed in a specific direction. Each pair of magnets generate a field with a different direction to that generated by the opposing pair of electromagnets, however, there will be an area (within the hypothetical circumference or sphere) through which the lines of all the fields generated by each pair of electromagnets pass, this is the intersection space between the fields. This space must coincide with the tumorous cellular aggregation that is to be eliminated from the animal organism.

If the device is formed of opposing magnetic fields it can contain four, six, eight, ten, twelve, fourteen, sixteen, etc. electromagnets, that is, an even number of electromagnets, so that they are all located on the perimeter of a hypothetical circumference or sphere and face each other in pairs. On the other hand, if each electromagnet acts alone the device can contain two, three, four or more electromagnets.

The intensity, time, direction and application rate of each electromagnetic field should be appropriately specified by, for example, the software program loaded into the device's microprocessor. The device contains a microprocessor equipped with a software program that determines the intensity of each electromagnetic field, the duration of the electromagnetic impulses and the application frequency of the magnetic fields.

### Description of the drawings

This descriptive report contains a selection of figures to accompany the description provided here and to help in the understanding of the present invention's characteristics as follows:
Figure 1 is a schematic view of the device incorporating the present invention and containing two pairs of electromagnets.
Figure 2 is a schematic view of the device incorporating the present invention and containing three pairs of electromagnets.
Figure 3 is a schematic view of the device incorporating the present invention and containing eight pairs of electromagnets.

### Preferred layout of the invention

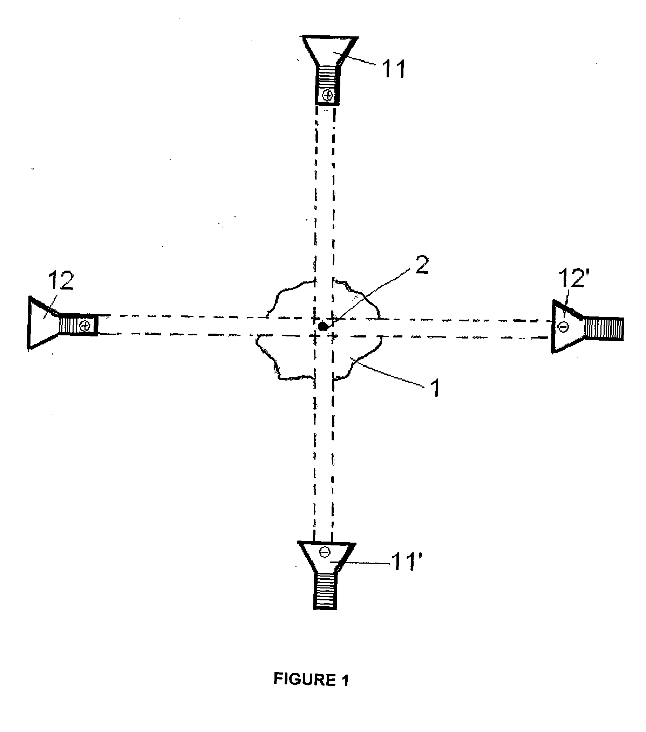

The device facilitates the selective destruction of cells, particularly those that present high cell division activity with uncontrolled proliferation in an animal organism. Its layout consists of at least two electromagnets whose axes form a specific angle, or at least four electromagnets that are simultaneously electrically activated and deactivated in pairs, having opposing poles and being axially paired and placed around a hypothetical circumference or sphere as shown in Figure 1. Each pair of facing magnets (11-11' and 12-12') generates an electromagnetic field in a physically predetermined direction. The space created at their intersection (2) can, therefore, be spatially predetermined and placed in a suitable position. This allows the location of the intersection space (2) to be altered using axial displacements (in the x, y and z axes) or by the movement of the equipment containing the electromagnets. The area to be treated (1) can, therefore, be precisely located in the intersection (2) of the magnetic circuits. Which, in turn, means that the area to be treated will always remain exposed to the physical effects provoked by the alternating variance of the activation of the magnetic fields that cross it. Figure 1 shows the magnetic fields generated by each pair of electromagnets (11 -11' and 12 -12') or (11 and 12) where an electromagnet, rather than a pair of opposing electromagnets, generates each field. It can be seen that there is a predetermined intersection space (2) whose position should correspond with the location of the cells to be destroyed (1) or treated.

Each pair of electromagnets correlated on the same axis (opposing) and generating an electromagnetic field are positioned so that their opposite poles (positive and negative) are seated facing each other. This means that the electromagnetic field is generated in the same orientation and direction as it passes through the cellular aggregation of interest (1) or material to be treated. This material will then experience the effect produced by the alternating variance of the fields generated in the coils when an electrical current passes through them.

The device includes a microprocessor (not shown) for the control of the device's parameters.

The electromagnets and the patient should be located so that the intersection space (2) coincides with the treatment area, that is, with the cellular aggregation to be treated (1). The magnetic fields are applied alternately and continuously in the following manner. One electromagnet (11), or opposing pair (11-11'), generates an electromagnetic field that orients the molecules in one direction. This field is then deactivated and the other electromagnet (12), or pair of electromagnets (12 -12'), is activated, generating a field in the other direction, which forces the bipolar molecules to rotate. This electromagnet (12), or pair of electromagnets (12 -12'), is then deactivated and the initial electromagnet (11), or pair (11-11'), is reactivated, causing the molecules to rotate again in line with the new field's orientation. This continues with a frequency sufficient to produce a more or less rapid heating caused by the movement of the bipolar molecules or polar cellular structure that reacts with the applied electromagnetic field. The preferred frequency is between 0.5 GHz and 10 GHz with a frequency of 915 MHz the optimal. The bipolar molecules that are affected by the magnetic fields rotate 90° (in the case represented in Figure 1) thereby producing friction and heating that leads to the death of the cell depending on the temperature reached and the length of time they remain at that temperature (for tumour cells the thermonecrotic temperature is 57°C with a dwell time of 1 second).

Obviously it is best if the location of the treatment area is known before treatment commences, in the case of a tumour it should be detected before the application of the variable electromagnetic fields.

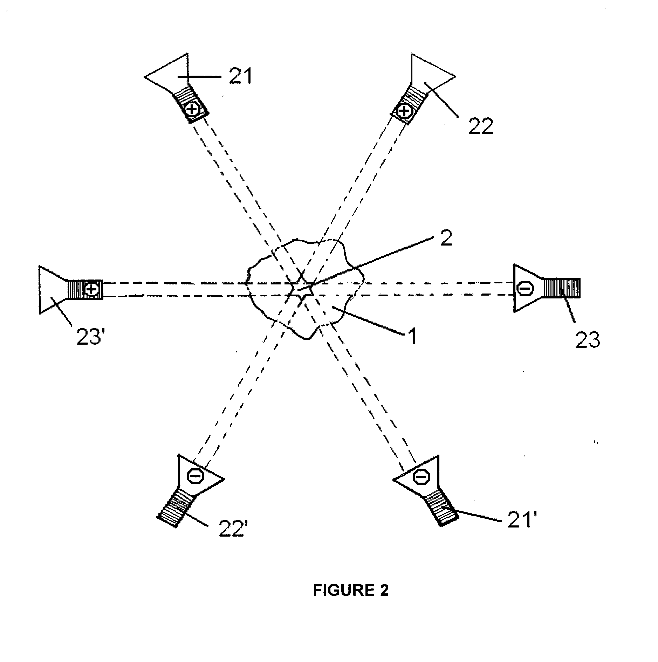

Figure 2 shows a similar device but it has six electromagnets arranged in opposing pairs. It is also possible to only use three electromagnets (21,22 and 23) that are not opposed. As previously stated, in this case the molecule rotation will be produced by the continuous and alternating application of the three magnetic fields. The method in this case will be as follows: an electromagnet (21), or a pair of opposing electromagnets (21-21') is initially activated (two opposing magnets provide greater certainty as, if one of them ceases functioning the device can continue to function). This activation generates an electromagnetic field that orientates the molecules in one direction. This field is then deactivated and another electromagnet (22), or a pair of opposing magnets (22-22'), is immediately activated. Thus generating a field in another direction forcing the bipolar molecules to rotate 60°, in this case. This electromagnet (22), or pair (22-22'), is then deactivated and the remaining electromagnet (23), or pair (23-23'), is activated so that the bipolar molecules rotate another 60° or 120° in relation to their previous position (the angle can be configured or selected). The electromagnet (23), or pair (23-23'), is then deactivated and the previous electromagnet (22), or pair (22-22'), is activated again, returning the molecules in question to their previous positions. This magnet (22), or pair (22-22'), is then deactivated and the initial magnet (21), or pair (21-21') is activated thereby returning the molecules to their initial position. The fields are maintained for sufficient time to achieve a necrotic temperature and dwell time sufficient to eliminate the cells of interest.

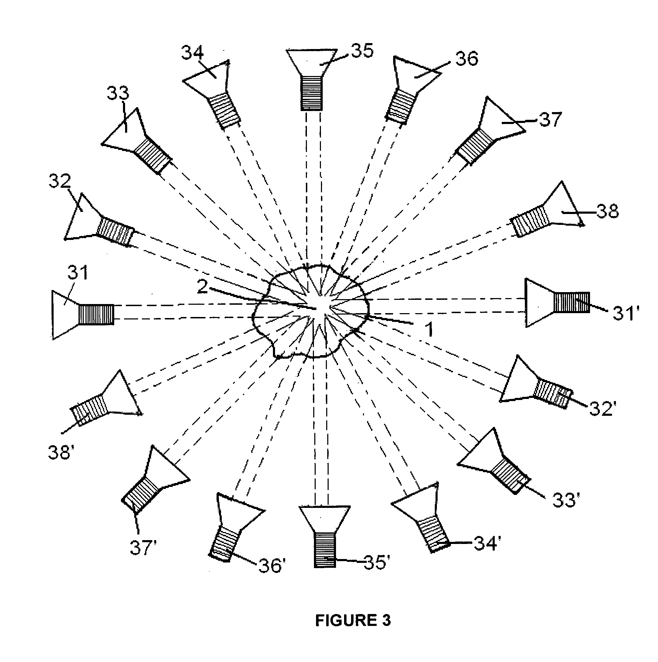

Figure 3 shows another example of the device with eight pairs of electromagnets (31-31'; 32-32'; 33-33'; 34-34'; 35-35'; 36-36'; 37-37' and 38-38'). The method to follow is similar to that explained above, but maintaining the same frequency of activation / deactivation (taking the reference pattern for the electromagnets with axes set at 90°). There is less thermal increase at lower angles and at greater angles there is a greater thermal increase. Shallower angles require an increase in frequency to achieve the same temperature increase in the bipole. If the frequency is not increased the progress or increase in temperature per unit time is less. For wider angles a lower frequency is required between activation and deactivation. The thermal effects produced by the electromagnetic fields in particular cells can be adjusted by varying this frequency. This provides the possibility of focussing the thermal effects as the temperature increase is affected by the composition or structure of the bipole and the orienting movement or "spin" of the bipole increases the friction with its surroundings, up to a maximum above which the friction coefficient decreases. This means that it is possible to maintain a predetermined temperature without surpassing a given value. However, if the strength of the magnetic field is increased (by increasing the ampage) the structural effects in the bipolar will increase. Eventually weakening the cohesion between its atoms thereby weakening or altering the behaviour of the cells of interest. This opens new possibilities as, if there are good cells in the focus area, they can be left unaffected and other cells can be killed. This means that the apparatus or equipment is selective and it will not produce cumulative side effects in the treated organism.

The intersection space (2) will be greater when the device contains a greater number of electromagnets. This implies that the area that can be treated will also increase as can be seen in Figures 1 to 3.

Another advantage distinguishable from the previous description is that the device permits a specific treatment with the objective of weakening or altering the structure of the tumour cells but leaving them alive so that they can be easily attacked by the immune system. This induces an "auto-vaccination" in the individual by indicating to the immune system the way to combat the new tumour cells that are being generated in the individual, this in turn means the individual will not have to be treated again for the same type of tumour.

A device can contain electromagnets with different coil diameters so that the field or focal volume can be varied by selecting different electromagnets. This means that a patient's treatment time can be minimised, as it will not depend on the size of the tumour to be treated.

## Claims

1. Method for the destruction of cells with uncontrolled proliferation in an animal organism, **characterised by** the following stages:
a) Detection of the cells with uncontrolled proliferation in the animal organism of interest.
b) Sequential and alternate application of at least two magnetic fields with distinct directions to the treatment area to achieve the heating and destruction of the cells, where each electromagnetic field is generated by at least one electromagnet, but preferably with a pair of electromagnets with their opposite poles opposing each other in order to better direct the flow lines.

2. Method for the destruction of cells with uncontrolled proliferation as in Claim 1 where the sequential and alternate application of the opposing magnetic fields is performed with a preferred frequency of between 0.5 GHz and 10 GHz.

3. Method for the destruction of cells with uncontrolled proliferation as in Claim 1 where before the sequential and alternate application of the opposing magnetic fields is performed the cells with uncontrolled proliferation are associated with paramagnetic or ferromagnetic materials to enhance the action of the electromagnetic fields.

4. Device for the destruction of cells with uncontrolled proliferation containing at least two electromagnets, where each one generates an electromagnetic field in one direction and orientation. The electromagnets are positioned so that the electromagnetic field lines generated by each magnet intersect in a predetermined or programmed spatial area.

5. Device for the destruction of cells with uncontrolled proliferation as in Claim 4, **characterised by** containing four or more electromagnets, an even number, located around the perimeter of a hypothetical circumference or sphere. Pairs of electromagnets face each other and these pairs generate an electromagnetic field in one direction and orientation.

6. Device for the destruction of cells with uncontrolled proliferation as in Claim 4 **characterised by** containing a microprocessor with a software program that determines the intensity, direction and frequency of each electromagnetic field generated.
